# EUROPEAN PATENT APPLICATION

(11) **EP 4 800 123 A1**
(43) Date of publication of application: **02.09.2026**
(21) Application number: 26160560.4
(22) Date of filing: 25.02.2026
(51) Int. Cl.: C12Q 1/6848, C12Q 1/686, C12Q 1/6876, C12Q 1/6851

(54) **INTERNAL CONTROL NUCLEIC ACID, METHOD FOR DESIGNING SAID NUCLEIC ACID, AND KIT COMPRISING SAID NUCLEIC ACID**

(30) Priority: 28.02.2025 JP 2025032288
(71) Applicant: Kanto Kagaku Kabushiki Kaisha, Tokyo 103-0023 (JP)
(72) Inventor: KONDO, Takumi, Kanagawa, 2591146 (JP); YAMAZAKI, Hiroyuki, Kanagawa, 2591146 (JP); SATO, Daichi, Kanagawa, 2591146 (JP); YOSHIDA, Masaki, Kanagawa, 2591146 (JP)
(74) Representative: Meissner Bolte Partnerschaft mbB

(57) **Abstract**

[Problem] The object of the present invention is to provide an internal control nucleic acid that can be used in samples from any biological system and that can accurately determine whether a target nucleic acid is positive or false negative, a method for designing said internal control nucleic acid, and a kit comprising said internal control nucleic acid.

[Solution] The present invention relates to an internal control nucleic acid having a unique base sequence that does not exist in nature, a method for designing said nucleic acid, and a kit comprising said nucleic acid.

## Description

### [Technical Field]

The present invention relates to an internal control nucleic acid used to determine whether an amplification reaction of a nucleic acid of a target gene has been carried out correctly during genetic testing, a method for designing said internal control nucleic acid, and a kit comprising said internal control nucleic acid.

### [Background Art]

When detecting a target nucleic acid using a nucleic acid amplification method, if the detection result is negative, it is important to determine whether it is a true negative (i.e., the target nucleic acid is not present in the sample) or a false negative (i.e., the target nucleic acid is present but not detected for some reason).

Reasons for false negatives include deterioration of the sample, defects in the process of extracting nucleic acids from the sample, the presence of inhibitors in the extracted nucleic acids, deterioration of the extracted nucleic acids, and defects in nucleic acid amplification reagents or equipment. One method for determining false negatives due to the presence of inhibitors in the extracted nucleic acid is to add an internal control nucleic acid, which has a base sequence that does not react with the primers and probes used to detect the test subject, to the sample, and then amplify and detect the internal control nucleic acid simultaneously with the target nucleic acid. According to this method, if the internal control nucleic acid is not detected, it can be determined that the result is a false negative due to inhibition of the amplification reaction. However, this method requires the design, synthesis, and confirmation of the reactivity of an internal control nucleic acid for each type of target nucleic acid, which is problematic in terms of time and cost. Therefore, there is a need for the development of a common internal control nucleic acid that is independent of the type of target nucleic acid, and a method for designing the same.

Patent Document 1 discloses an internal control nucleic acid molecule that comprises at least one forward primer binding site, at least one reverse primer binding site, and at least one amplifiable region, all of which are randomly generated, and also discloses a method for designing the same. However, although the internal control nucleic acid described in this document is edited to a desired length by joining 10 random sequences, each about 50 bases in length, no guidelines are disclosed, such as specific selection criteria for the designing method such as E-value, or specific criteria for acceptable sequence homology between the obtained sequence and a known sequence. Therefore, there remains a need to develop a method for easily and reproducibly designing sequences that have sufficiently low homology to known sequences and are independent of the type of target nucleic acid.

### [Prior Art Documents]

### [Patent Documents]

[Patent Document 1] Japanese Patent No. 4805158

### [Summary of the Invention]

### [Problem to Be Solved by the invention]

The object of the present invention is to provide an internal control nucleic acid that can be used in samples from any biological system and that can accurately determine whether a target nucleic acid is positive or false negative, a method for designing the internal control nucleic acid, and a kit comprising the internal control nucleic acid.

### [Means for Solving the Problem]

The present inventors have recognized that a common internal control nucleic acid must have a base sequence that is not thought to be contained in a sample, and have discovered that the above problem can be solved by using an E-value to design an internal control nucleic acid having a unique base sequence that does not exist in nature, and that the use of an internal control nucleic acid having a unique base sequence that does not exist in nature, in a nucleic acid amplification method for detecting a target nucleic acid enables to determine whether or not a result is a false negative; after repeated trial and error, the present inventors have completed the present invention.

The present invention relates to the following inventions.
[1] An internal control nucleic acid comprising a sequence that is at least 90% identical to a sequence of 50 or more consecutive bases selected from the base sequence shown in SEQ ID NO: 1.
[2] The internal control nucleic acid according to [1], wherein the internal control nucleic acid comprises a sequence of 50 or more consecutive bases selected from the base sequence shown in SEQ ID NO: 1.
[3] The internal control nucleic acid according to [1], wherein the internal control nucleic acid consists of a sequence of 50 or more consecutive bases selected from the base sequence shown in SEQ ID NO: 1.
[4] The internal control nucleic acid according to [1], wherein the internal control nucleic acid consists of the base sequence shown in SEQ ID NO: 1.
[5] A composition for the detection and/or quantification of nucleic acids, containing:
   an internal control nucleic acid according to any of [1] to [4];
   a forward primer and a reverse primer for amplifying a part or all of the nucleic acid; and
   a sample.
[6] The composition according to [5], further containing an oligonucleotide probe complementary to an internal control nucleic acid in the region flanked by the forward primer and the reverse primer.
[7] The composition according to [5], wherein the oligonucleotide of the forward primer is at least 95% identical to the base sequence shown in SEQ ID NO: 2.
[8] The composition according to [5], wherein the oligonucleotide of the reverse primer is at least 95% identical to the base sequence shown in SEQ ID NO: 3.
[9] The composition according to [3], wherein the oligonucleotide probe is at least 95% identical to the base sequence shown in SEQ ID NO: 4.
[10] A method for detecting and/or quantifying a nucleic acid in a sample, comprising:
   a) a step of mixing an internal control nucleic acid comprising a sequence that is at least 90% identical to 50 or more consecutive bases selected from the base sequence shown in SEQ ID NO: 1 with a forward primer and a reverse primer for amplifying a part or all of the nucleic acid, and a sample;
   b) a step of extending the forward and reverse primers, thereby generating at least one target amplicon; and
   c) a step of detecting and/or quantifying the amplification of the target amplicon by electrophoresis.
[11] A method for detecting and/or quantifying a nucleic acid in a sample, comprising:
   a) a step of mixing an internal control nucleic acid comprising a sequence that is at least 90% identical to 50 or more consecutive bases selected from the base sequence shown in SEQ ID NO: 1 with a forward primer and a reverse primer for amplifying a part or all of the nucleic acid, an oligonucleotide probe, and a sample;
   b) a step of extending the forward and reverse primers, thereby generating at least one target amplicon;
   c) a step of binding the oligonucleotide probe to said at least one internal control nucleic acid or target amplicon; and
   d) a step of detecting a signal proportional to the amount of said at least one internal control nucleic acid or target amplicon.
[12] The method according to [11], wherein the internal control consists of a sequence of 50 or more consecutive bases selected from the base sequence shown in SEQ ID NO: 1.
[13] The method according to [12], wherein the internal control comprises a sequence consisting of the base sequence shown in SEQ ID NO: 1.
[14] The method according to [10] or [11], wherein the oligonucleotide of the forward primer is at least 95% identical to the base sequence shown in SEQ ID NO: 2.
[15] The method according to [10] or [11], wherein the oligonucleotide of the reverse primer is at least 95% identical to the base sequence shown in SEQ ID NO: 3.
[16] The method according to [10] or [11], wherein the oligonucleotide of the forward primer is the base sequence shown in SEQ ID NO: 2, and the oligonucleotide of the reverse primer is the base sequence shown in SEQ ID NO: 3.
[17] The method according to [10] or [11], wherein the internal control nucleic acid comprises a sequence of 50 or more consecutive bases selected from the base sequence shown in SEQ ID NO: 1.
[18] The method according to [10] or [11], wherein the internal control nucleic acid consists of a sequence of 50 or more consecutive bases selected from the base sequence shown in SEQ ID NO: 1.
[19] The method according to [11], wherein the oligonucleotide probe is the base sequence shown in SEQ ID NO: 4.
[20] The method according to [10] or [11], further comprising a step of extracting a nucleic acid from the sample before or after step a).
[21] A method for designing an internal control nucleic acid in the detection and/or quantification of a nucleic acid, comprising:
   a) a step of generating a random base sequence having a length of 500 to 1500 bases; and
   b) a step of comparing the random base sequence with a database to select sequences with low homology, wherein the comparison with the database is performed using BLAST, and the E-value of the BLAST selection criteria is E-value>30.
[22] The method according to [21], further comprising a step of joining regions with high E-values from one of the base sequences selected in step b), or
   a step of joining regions with high E-values from the two or more base sequences selected in b).
[23] The method according to [21], further comprising a step of removing a base sequence containing five or more consecutive identical bases from the base sequences selected in b).
[24] The method according to [21], further comprising a step of adjusting the GC content of the base sequence selected in b) to 40 to 60%.
[25] The method according to any of [21] to [24], wherein the designed internal control nucleic acid has an E-value of 300 or more by BLAST and a length of 500 to 1500 bases.
[26] A method for designing an internal control nucleic acid in the detection and/or quantification of a nucleic acid, comprising:
   a) a step of generating a random base sequence having a length of 500 to 1500 bases;
   b) a step of selecting a sequence with low homology from the random base sequences by comparing with a database, wherein the comparison with the database is performed using BLAST, and the E-value of the BLAST selection criteria is E-value>30;
   c) a step of removing a base sequence containing five or more consecutive identical bases from the base sequences selected in b);
   d) a step of adjusting the GC content of the base sequence selected in b) to 40-60%; and/or
   e) a step of further selecting a region with a high E-value from one base sequence selected in b) and joining the selected regions, or a step of selecting a region with a high E-value from two or more base sequences selected in b) and joining the selected regions;
   wherein the designed internal control nucleic acid has an E-value of 300 or more by BLAST and a length of 500 to 1500 bases.

### [Effects of the Invention]

The internal control nucleic acid of the present invention has low homology to known nucleic acid sequences and can therefore be used as an internal control in nucleic acid amplification methods for detecting various genes regardless of the specimen type (animal, plant, microorganism, virus). Furthermore, whereas it has previously been necessary to prepare an internal control nucleic acid and a primer/probe for detecting the internal control nucleic acid for each specimen type and target gene to be detected, the present invention makes it possible to standardize the internal control nucleic acid and the primer/probe for detecting the internal control nucleic acid, thereby reducing the development time and cost of genetic testing kits. The internal control nucleic acid of the present invention can be used to distinguish between true negatives and false negatives, and to confirm that the nucleic acid amplification reaction proceeded without any problems.

### [Brief Description of the Drawings]

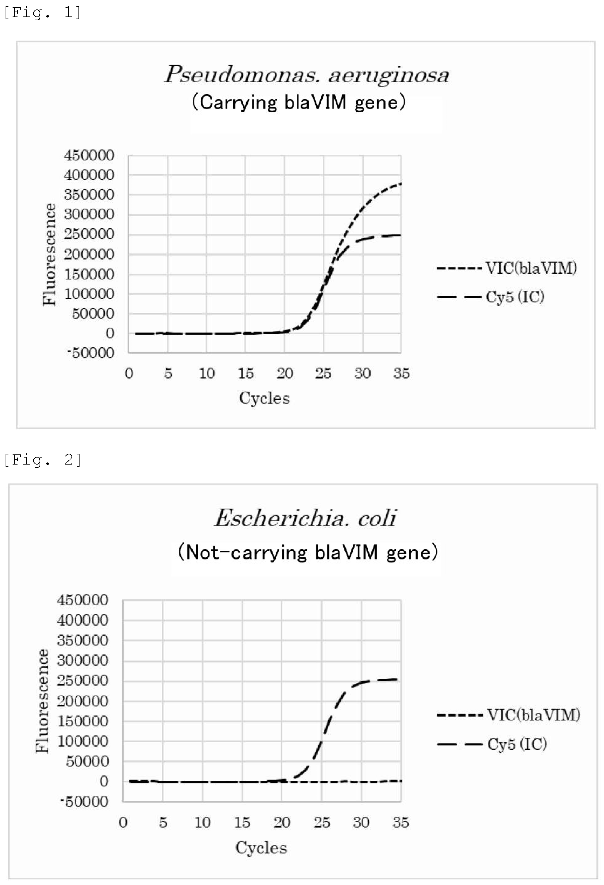

[FIG. 1] FIG. 1 shows the results of real-time PCR detection of a PCR reaction solution prepared containing DNA of *Pseudomonas aeruginosa* (carrying the blaVIM gene) as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 5, a reverse primer of SEQ ID NO: 6, and an oligonucleotide probe of SEQ ID NO: 7.
[FIG. 2] FIG. 2 shows the results of real-time PCR detection of a PCR reaction solution prepared containing DNA of *Escherichia coli* (not carrying the blaVIM gene) as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 5, a reverse primer of SEQ ID NO: 6, and an oligonucleotide probe of SEQ ID NO: 7.

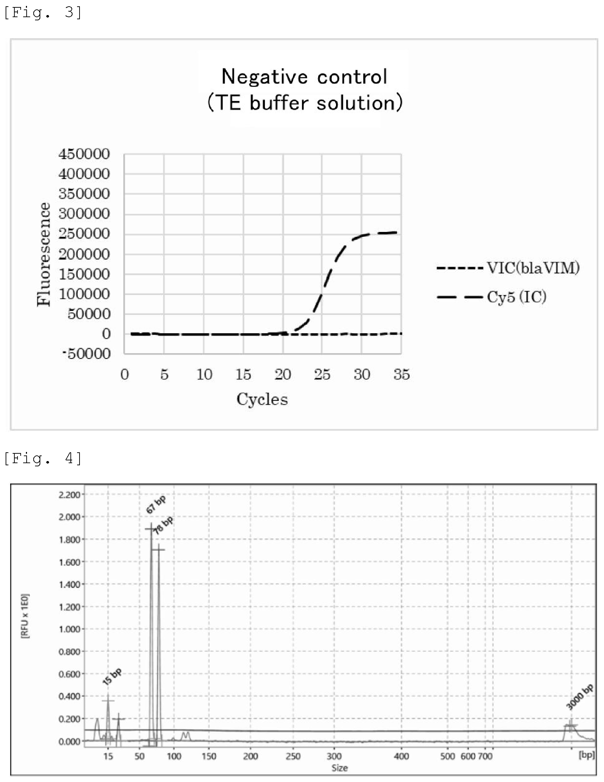

[FIG. 3] FIG. 3 shows the results of real-time PCR detection of a PCR reaction solution prepared containing TE buffer solution as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 5, a reverse primer of SEQ ID NO: 6, and an oligonucleotide probe of SEQ ID NO: 7.
[FIG. 4] FIG. 4 shows the results of analyzing a PCR reaction solution containing DNA of *Pseudomonas aeruginosa* (carrying the *bla*VIM gene) as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 5, a reverse primer of SEQ ID NO: 6, and an oligonucleotide probe of SEQ ID NO: 7, which was prepared, subjected to real-time PCR, and then subjected to capillary electrophoresis of the sample, with the fluorescence intensity on the vertical axis and the size (bp: base pairs) on the horizontal axis of the graph.

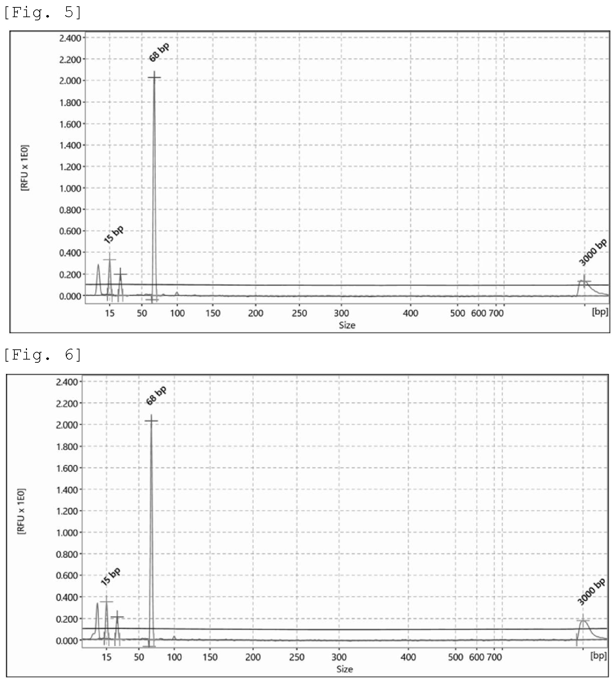

[FIG. 5] FIG. 5 shows the results of analyzing a PCR reaction solution containing DNA of *Escherichia coli* (not carrying the *bla*VIM gene) as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 5, a reverse primer of SEQ ID NO: 6, and an oligonucleotide probe of SEQ ID NO: 7, which was prepared, subjected to real-time PCR, and then subjected to capillary electrophoresis of the sample, with the fluorescence intensity on the vertical axis and the size (bp: base pairs) on the horizontal axis of the graph.
[FIG. 6] FIG. 6 shows the results of analyzing a PCR reaction solution containing TE buffer solution as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 5, a reverse primer of SEQ ID NO: 6, and an oligonucleotide probe of SEQ ID NO: 7, which was prepared, subjected to real-time PCR, and then subjected to capillary electrophoresis of the sample, with the fluorescence intensity on the vertical axis and the size (bp: base pairs) on the horizontal axis of the graph.

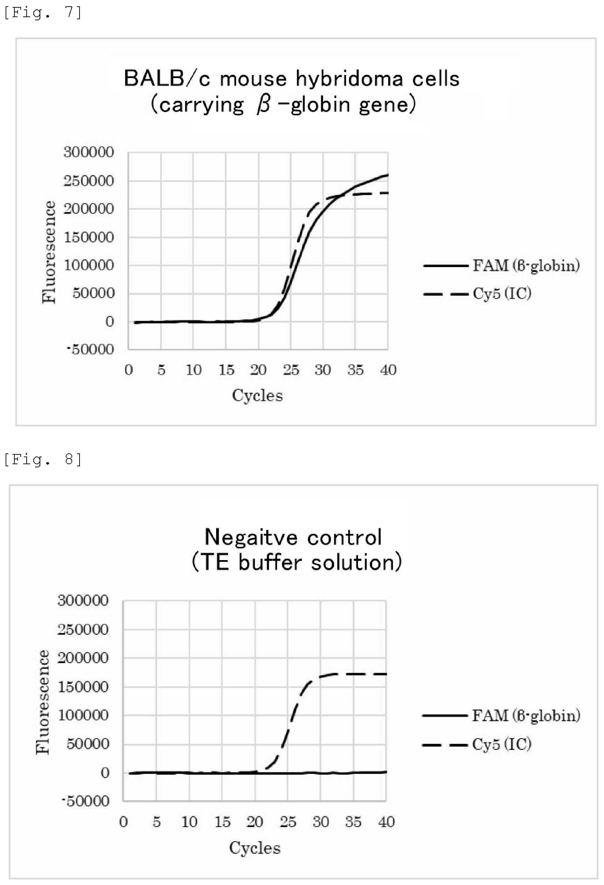

[FIG. 7] FIG. 7 shows the results of real-time PCR detection of a PCR reaction solution prepared containing DNA of mouse hybridoma cells (carrying the β-globin gene) as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 8, a reverse primer of SEQ ID NO: 9, and an oligonucleotide probe of SEQ ID NO: 10.
[FIG. 8] FIG. 8 shows the results of real-time PCR detection of a PCR solution prepared containing TE buffer solution as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 8, a reverse primer of SEQ ID NO: 9, and an oligonucleotide probe of SEQ ID NO: 10.

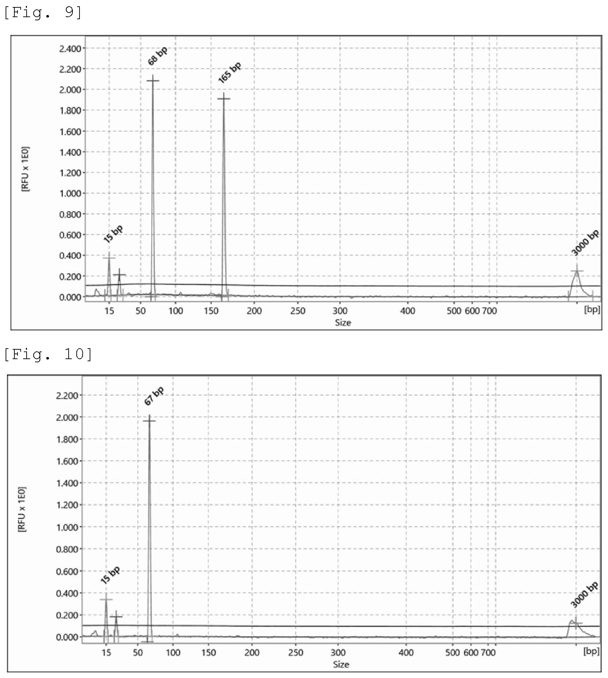

[FIG. 9] FIG. 9 shows the results of analyzing a PCR reaction solution prepared containing DNA of mouse hybridoma cells (carrying the β-globin gene) as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 8, a reverse primer of SEQ ID NO: 9, and an oligonucleotide probe of SEQ ID NO: 10, which was prepared, subjected to real-time PCR, and then subjected to capillary electrophoresis of the sample, with the fluorescence intensity on the vertical axis and the size (bp: base pairs) on the horizontal axis of the graph.
[FIG. 10] FIG. 10 shows the results of analyzing a PCR reaction solution prepared containing TE buffer solution as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 8, a reverse primer of SEQ ID NO: 9, and an oligonucleotide probe of SEQ ID NO: 10, which was prepared, subjected to real-time PCR, and then subjected to capillary electrophoresis of the sample, with the fluorescence intensity on the vertical axis and the size (bp: base pairs) on the horizontal axis of the graph.

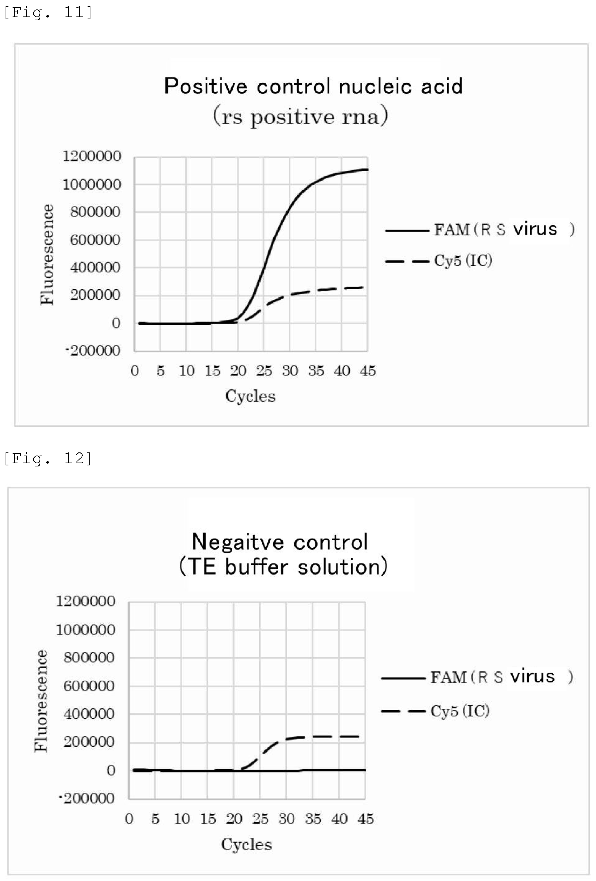

[FIG. 11] FIG. 11 shows the results of real-time RT-PCR detection of an RT-PCR reaction solution containing an RS virus positive control nucleic acid of SEQ ID NO: 11 as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 12, a reverse primer of SEQ ID NO: 13, and an oligonucleotide probe of SEQ ID NO: 14.
[FIG. 12] FIG. 12 shows the results of real-time RT-PCR detection of an RT-PCR solution prepared containing TE buffer solution as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 12, a reverse primer of SEQ ID NO: 13, and an oligonucleotide probe of SEQ ID NO: 14.

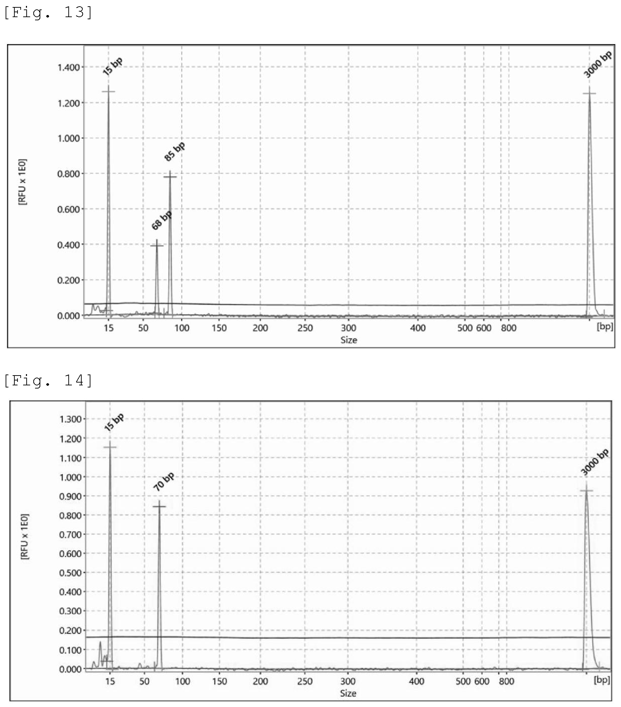

[FIG. 13] FIG. 13 shows the results of analyzing an RT-PCR reaction solution containing an RS virus positive control nucleic acid of SEQ ID NO: 11 as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 12, a reverse primer of SEQ ID NO: 13, and an oligonucleotide probe of SEQ ID NO: 14, which was prepared, subjected to real-time PCR, and then subjected to capillary electrophoresis of the sample, with the fluorescence intensity on the vertical axis and the size (bp: base pairs) on the horizontal axis of the graph.
[FIG. 14] FIG. 14 shows the results of analyzing an RT-PCR reaction solution containing TE buffer solution as a sample, an internal control nucleic acid of SEQ ID NO: 1, a forward primer of SEQ ID NO: 2, a reverse primer of SEQ ID NO: 3, an oligonucleotide probe of SEQ ID NO: 4, a forward primer of SEQ ID NO: 12, a reverse primer of SEQ ID NO: 13, and an oligonucleotide probe of SEQ ID NO: 14, which was prepared, subjected to real-time PCR, and then subjected to capillary electrophoresis of the sample, with the fluorescence intensity on the vertical axis and the size (bp: base pairs) on the horizontal axis of the graph.

### [Detailed Description of the Invention]

The internal control nucleic acid of the present invention is used to accurately determine whether a target nucleic acid is positive or false negative in the amplification reaction of a target gene in a sample.

The sample is not particularly limited and may be any biological sample. The sample may be a sample derived from, for example, animal, plant, microorganism, virus, or the like.

### [Internal control nucleic acid]

One aspect of the present invention relates to provide an internal control nucleic acid having a unique base sequence that does not exist in nature, which is artificially created to have low homology to known nucleic acid sequences and comprises at least one forward primer binding site, at least one reverse primer binding site, and at least one amplifiable region. The internal control nucleic acid of the present invention may also comprise a probe binding site.

In one aspect of the invention, there is provided an internal control nucleic acid comprising or consisting of a sequence that is at least 80%, 85%, 90%, 91%, 92%, 93%, 94%, 95%, 96%, 97%, 98%, 99% or more identical to a sequence of 50 to 1000 consecutive bases selected from the base sequence shown in SEQ ID NO: 1. In one embodiment, the internal control nucleic acid comprises or consists of a sequence of consecutive 50 bases or more, 60 bases or more, 70 bases or more, 80 bases or more, 90 bases or more, 100 bases or more, 150 bases or more, 200 bases or more, 250 bases or more, 300 bases or more, 350 bases or more, 400 bases or more, 450 bases or more, 500 bases or more, 550 bases or more, 600 bases or more, 650 bases or more, 700 bases or more, 750 bases or more, 800 bases or more, 850 bases or more, 900 bases or more, 950 bases or more, or more selected from the base sequence shown in SEQ ID NO: 1. In a preferred embodiment, the internal control nucleic acid consists of the base sequence shown in SEQ ID NO:1.

The internal control nucleic acid of the present invention includes, but is not limited to, the following sequence (E-value: 348 (as of November 7, 2024)):

GCACATGTGC TGACCCAGAT TAGACGTGAG ATGAAGGTCT TGTAAACCAG GTCAAGTACT 60
CTTGAAGTGA CACGATCCAC GATGTCCACC GTTGGGACGT GAGTTCCAAC GGTGTAAGGT 120
GAAGATCCGT GACTTCTCAT GGAAGCACGA TGGACTTGCA CATATCTATT CTGACACGGA 180
GTCTAGAGGA GCTAGTCTCT TAGGTGCGAC CAGGATTAGT CCCAACATTA CGGGAGTCGT 240
CAATTAGACG TCTATCCATG GCAGAATCCT AATGGGTACC GAGATGGAGT CGAACCTCTC 300
TAGTCCTAAG CTCGAGAGCG ATGGTTCTAT CGGATTCTGC TACCTATGAT CGTAAGGACT 360
TAATACGCTG GGGTCAACTA GTAATGTGCC AGTGGCATAG TTTCATGCAG ATACTGCTCC 420
GCTAAGTGGC TCAAGGGTCA GTTGATATCT TGCACCGTTC AGCCATCTCC CTAGCATGCA 480
CACAAAGTTC TCTTATGTAC GGACTTCGCC ACGAACTAAC AATCTGGTGT AGCATCA|TCC 540
ACTGACGCGA ATGCATTCCG CTATCATCAC CATATAGTGG TGCTTGTATG AGGTCAAGTC 600
CCTGATTAAT CCGCATAAGC AATCCTTTGG GCTACACCCA GTTAGATTAG CGATCAGGGA 660
TGTATTCATT CATGAGTAGG CTAGTGGCTG GAACTTCTAT GCCCATCTAC GTCCTCTACA 720
AGACAGCTCG TCATAAGTTG AGTATACGGA CCGGACCAAG ATTGGATTGC TCACTGGTCG 780
CGATGTGCAA TCTCTACCGA GTTTCTCGTA CCGAAGGTAC GATCTTCCCA TAGACGCATA 840
CATCGAGGAT CGAACTGCCT TAAATGACGG AACGCCATTA CTAGGCTCAT CGACGCGCTA 900
CGGTATGTTC AACTCAGGAG ACCTTCTCTA CCCATCGTTA ACGTGCTGAA CTAATCAGGT 960
TCGACAATCT CGATTGATAC ACGGATCGGA ATTGGCTTAG 1000
[SEQ ID NO: 1]

### [Method for designing internal control nucleic acids]

Another aspect of the present invention relates to provide methods for designing internal control nucleic acids.

One aspect of the present invention provides a method for designing an internal control nucleic acid in the detection and/or quantification of a nucleic acid. Such a designing method typically involves:
a) a step of generating a random base sequence having a length of 500 to 1500 bases, wherein the "a) step of generating" may be performed using computer software;
b) a step of comparing the random base sequence with a database to select sequences with low homology, wherein the comparison with the database is performed using BLAST, and the BLAST selection criterion is an E-value of 30 or more.

Here, the length of the random base sequence of a) may be 500 to 1500 bases, preferably 600 to 1400 bases, 700 to 1300 bases, 800 to 1200 bases, or more preferably 900 to 1100 bases. The E-value of the BLAST selection criteria in b) may be 0.1 or more, 1 or more, 2 or more, 3 or more, 4 or more, 5 or more, 6 or more, 7 or more, 8 or more, 9 or more, 10 or more, 20 or more, 30 or more, 40 or more, 50 or more, 100 or more, or more. The E-value of the selection criteria may be 20 or less, 30 or less, 40 or less, 50 or less, 100 or less, 200 or less, 300 or less, 400 or less, 500 or less, 600 or less, 700 or less, 800 or less, 900 or less, or 1000 or less. The upper and lower limits can be appropriately selected and such ranges can be used as the selection criteria.

The internal control nucleic acid of the present invention can be artificially designed.

By artificially, it is meant that the sequences are randomly designed while taking into account certain design characteristics. The internal control nucleic acid can be determined by any method for determining a random base sequence of four nucleic acid bases (A, T, G, and C). Examples of such methods include, but are not limited to, using computer software designed to generate random base sequences. Any software for text editing can be used as such computer software. Examples of software for text editing include Python's random module, random character generator software, and PIN generation software.

In one embodiment of the present invention, the random base sequence is generated at least once, for example, 5 times or more, 10 times or more, 50 times or more, 100 times or more, 500 times or more, 1000 times or more, 5000 times or more, or 10,000 times or more. From the random base sequences, base sequences with low homology are selected by comparison with a database. Such comparison with databases may be performed using BLAST, such as nucleotide BLAST (blastn). BLAST is basically a tool for searching for low E-values (high homology), and in the default settings, E-values of 10 or less are displayed. In the present invention, the lower limit of the E-value in the selection criteria of blastn is selected to be a higher value than usual, and may be, for example, 1, 2, 3, 4, 5, 6, 7, 8, 9, 10, 20, 30, 40, 50, 60, 70, 80, 90, 100 or more. In the present invention, the upper limit (cutoff value) of the E-value in the selection criteria of blastn is usually selected to be a value higher than the default setting (10), and may be, for example, 20, 30, 40, 50, 60, 70, 80, 90, 100, 200, 300, 400, 500, 600, 700, 800, 900, 1000, or 2000. In a preferred embodiment of the present invention, the E-value of the selection criteria is 30. In a preferred embodiment, the E-value of the selection criteria may be 30 to 1,000. In the present invention, the above lower limit and upper limit can be appropriately selected and such ranges can be used as the selection criteria. Since E-values fluctuate daily, the above figures should be considered as examples and are not limiting.

The method of designing an internal control nucleic acid in the present invention may comprise a step of further processing the sequence obtained using computer software designed to generate a random base sequence. For example, regions with high E-values in the sequences obtained using computer software may be selected and joined together, or multiple sequences with high E-values may be selected from the sequences obtained using computer software and combined. Furthermore, the designing method may include a step of removing consecutive bases or sequences from the selected base sequence, and the consecutive bases are 4 or more bases, 5 or more bases, 6 or more bases, 7 or more bases, 8 or more bases, 9 or more bases, 10 or more bases, or more. Here, a high E-value means that the E-value is 1 or more, 2 or more, 3 or more, 4 or more, 5 or more, 6 or more, 7 or more, 8 or more, 9 or more, 10 or more, 20 or more, 30 or more, or more, but is not limited to these. Processing methods include, but are not limited to, a method using computer software. Any software for gene analysis and text editing can be used as such computer software.

The homology of the internal control nucleic acid of the present invention to a known or natural sequence is determined by analysis using BLAST (Basic Local Alignment Search Tool), and the nucleic acid is prepared so that the E-value is 100 or more, 200 or more, and preferably 300 or more. The E-value (Expectation value) is the expected value when the sequence happens to be in the database. An E-value of 1 indicates that there is one sequence in the database. The higher the homology, the smaller the E-value, and the lower the homology, the larger the E-value. As a guideline, if the E-value is smaller than 0.0001, it is considered that there is homology. As the number of sequences registered in the database increases, it is expected that the E-value will become smaller in the future, even for the same sequence. In general, the E-value is thought to decrease as the database is updated, so the E-value is not necessarily limited to the range described above, and the upper and lower limits of the E-value and the ranges therebetween may become smaller.

The internal control nucleic acid of the present invention is preferably designed so as not to comprise a repeated region of identical base pairs of 10 bases or more, 9 bases or more, 8 bases or more, 7 bases or more, 6 bases or more, 5 bases or more, or 4 bases or more within its sequence. In a preferred embodiment of the present invention, the internal control nucleic acid is prepared so as not to comprise a repeated region of 5 or more identical base pairs. Furthermore, the method for designing an internal control nucleic acid in the present invention may comprise adjusting the GC content in the sequence.

The method for designing an internal control nucleic acid may further comprise c) a step of removing a base sequence containing five or more consecutive identical bases from the base sequences selected in b).

The method for designing an internal control nucleic acid may further comprise d) a step of adjusting the GC content in the base sequence selected in b). The GC content after step d) may be from 20% to 80%, preferably from 30% to 70%, more preferably from 40 to 60%. In one embodiment of the invention, the GC content after step d) is adjusted to 48%.

The method for designing an internal control nucleic acid may further comprise: e) a step of selecting a region with a high E-value from one base sequence selected in b) and joining the selected regions, or a step of selecting a region with a high E-value from two or more base sequences selected in b) and joining the selected regions. Here, a high E-value means, but is not limited to, 1 or more, 2 or more, 3 or more, 4 or more, 5 or more, 6 or more, 7 or more, 8 or more, 9 or more, 10 or more, 20 or more, 30 or more, or more. Here, the minimum E-value by BLAST of the designed internal control nucleic acid may be, for example, 30, 40, 50, 60, 70, 80, 90, 100, 150, 200, 250, 300, 350, or 400. The maximum E-value by BLAST of the designed internal control nucleic acid may be, for example, 450, 500, 550, 600, 650, 700, 750, 800, 850, 900, 950, 1000 or more. In a preferred embodiment, the designed internal control nucleic acid has an E-value of 300 or more by BLAST and a length of 500 to 1500 bases.

### [Method for amplifying, detecting, and quantifying nucleic acids using internal control nucleic acids]

In one aspect of the invention, a method for detecting and/or quantifying a nucleic acid in a sample is provided. In the method, the following steps may be comprised: a) a step of mixing an internal control nucleic acid comprising or consisting of a sequence at least 90% or more, 91% or more, 92% or more, 93% or more, 94% or more, 95% or more, 96% or more, 97% or more, 98% or more, 99% or more, or more identical to a sequence of consecutive 50 bases or more, 60 bases or more, 70 bases or more, 80 bases or more, 90 bases or more, 100 bases or more, 150 bases or more, 200 bases or more, 250 bases or more, 300 bases or more, 350 bases or more, 400 bases or more, 450 bases or more, 500 bases or more, 550 bases or more, 600 bases or more, 650 bases or more, 700 bases or more, 750 bases or more, 800 bases or more, 850 bases or more, 900 bases or more, 950 bases or more, or more and 1000 bases or less selected from the base sequence shown in SEQ ID NO: 1, with a forward primer, a reverse primer for amplifying a part or all of the nucleic acid, and a sample; b) a step of extending the forward and reverse primers, thereby generating at least one target amplicon; and c) a step of detecting and/or quantifying the amplification of the target amplicon by electrophoresis. In a preferred embodiment, the internal control nucleic acid consists of the base sequence shown in SEQ ID NO: 1.

The generation and amplification of the target amplicon is not particularly limited, and known nucleic acid amplification methods can be applied. Examples of such methods include PCR, RT-PCR (Reverse Transcription-Polymerase Chain Reaction), LAMP (Loop-mediated Isothermal Amplification), RT-LAMP (Reverse Transcription-loop mediated isothermal amplification of DNA), TMA (Transcription Mediated Amplification), NASBA (Nucleic Acid Sequence-Based Amplification), SDA (Strand Displacement Amplification), and ICAN (Isothermal and Chimeric primer-initiated Amplification of Nucleic acids), etc. For each nucleic acid amplification method, those skilled in the art can select appropriate conditions using conventional means in the technical field.

The detection of the amplified target amplicon is not particularly limited, and can be carried out by determining the presence or absence of amplified nucleic acid by known methods. Such known detection methods include, for example, agarose gel electrophoresis, a method of measuring fluorescence using an intercalator, a method of measuring fluorescence using a fluorescently labeled probe, and a method of detecting the turbidity of magnesium pyrophosphate generated during nucleic acid amplification.

The quantification of the amplified target amplicon is not particularly limited, and can be carried out by measuring the amount of the amplified nucleic acid by a known method. Such known quantification methods include, for example, agarose gel electrophoresis, a method of measuring fluorescence using an intercalator, a method of measuring fluorescence using a fluorescently labeled probe, and a method of quantification based on the turbidity of magnesium pyrophosphate generated during nucleic acid amplification.

Since the internal control nucleic acid has low homology with other sequences, it is possible to design primers at any position in the internal control nucleic acid, thereby changing the region and length to be amplified. Furthermore, the length of the original internal control nucleic acid can be selected arbitrarily according to the region to be amplified. Furthermore, in order to design an internal control nucleic acid of a desired size, primers may be selected based on the desired size of the amplifiable region. For example, the primers described in the section "Kit comprising internal control nucleic acid" below can be used.

In a preferred embodiment, the method further comprises a step of extracting a nucleic acid from the sample before or after step a). By undergoing such an extraction step, there is an advantage that the subsequent PCR reaction can be made more efficient.

In a preferred embodiment of the present invention, the standard two-step PCR conditions comprise the following temperature cycles: (i) 94°C to 98°C for 5 to 300 seconds (thermal denaturation of template double-stranded DNA);
(ii) 55°C to 72°C, 5 seconds to 180 seconds (primer annealing and DNA extension, probe degradation and fluorescence detection) (25 to 50 cycles of (i) and (ii)).
The standard three-step PCR conditions comprise the following temperature cycles: (i) 94°C to 98°C for 5 to 300 seconds (thermal denaturation of template double-stranded DNA);
(ii) 55°C to 65°C, 5 seconds to 60 seconds (primer annealing);
(iii) 72°C, 5 seconds to 180 seconds (DNA elongation, fluorescence detection) (25 to 50 cycles of (i), (ii), and (iii)).

Another aspect of the present invention provides a method for detecting and/or quantifying a nucleic acid in a sample, the method comprising:
a) a step of mixing an internal control nucleic acid comprising or consisting of a sequence at least 90% or more, 91% or more, 92% or more, 93% or more, 94% or more, 95% or more, 96% or more, 97% or more, 98% or more, 99% or more, or more identical to a sequence of consecutive 50 bases or more, 60 bases or more, 70 bases or more, 80 bases or more, 90 bases or more, 100 bases or more, 150 bases or more, 200 bases or more, 250 bases or more, 300 bases or more, 350 bases or more, 400 bases or more, 450 bases or more, 500 bases or more, 550 bases or more, 600 bases or more, 650 bases or more, 700 bases or more, 750 bases or more, 800 bases or more, 850 bases or more, 900 bases or more, 950 bases or more, or more and 1000 bases or less selected from the base sequence shown in SEQ ID NO: 1, with a forward primer, a reverse primer for amplifying a part or all of the nucleic acid, an oligonucleotide probe, and a sample;
b) a step of extending the forward and reverse primers, thereby generating and amplifying at least one target amplicon;
c) a step of binding the oligonucleotide probe to said at least one internal control nucleic acid or target amplicon; and
d) a step of detecting a signal proportional to the amount of said at least one internal control nucleic acid or target amplicon.
Also in a preferred embodiment, the method further comprises a step of extracting a nucleic acid from the sample before or after step a). In a preferred embodiment, the internal control nucleic acid consists of the base sequence shown in SEQ ID NO: 1.

In c), the internal control nucleic acid is bound to the oligonucleotide probe at a probe binding region constructed complementary to the oligonucleotide probe. In c), the target amplicon binds to the oligonucleotide probe by complementarity. The internal control nucleic acid molecule may also comprise at least one probe binding region. The probe binding region is constructed to be complementary to the oligonucleotide probe. The probe for the complementary probe binding region may be any probe commonly known and utilized in detection and/or quantification assays for nucleic acids (DNA and/or RNA). The nucleotide sequence of the probe may be designed to bind to the complementary strand of the internal control nucleic acid.

In real-time PCR, PCR amplification products are detected by fluorescence. There are two types of fluorescence detection methods: a method using an intercalator and a method using a fluorescently labeled probe. For example, the probes described in the "Kit comprising internal control nucleic acid" below can be used.

d) Detection of the signal is carried out by means conventional in the art, such as tracking the fluorescence intensity over time. Intercalators include SYBR Green I, TB Green, ResoLight, and the like. Fluorescent dyes include, for example, FAM, HEX, VIC, ROX, Red610, and Cy5. Quencher dyes include, for example, TAMRA, BHQ-1, BHQ-2, BHQ-3, and the like.

### [Kit comprising internal control nucleic acid]

Another aspect of the present invention relates to a kit for determining false-negative results of a target nucleic acid, which kit comprises the internal control nucleic acid of the present invention and a primer pair for amplifying the internal control nucleic acid. The internal control nucleic acid may be any of those described above in [Internal control nucleic acid]. The kit may include instructions.

Examples of forward primer sequences that can be used to amplify the internal control nucleic acid include, but are not limited to, the following:
TAAGTGGCTC AAGGGTCAG [SEQ ID NO: 2]

Examples of reverse primer sequences that can be used to amplify the internal control nucleic acid include, but are not limited to, the following:

### CCTAGCATGC ACACAAAGTT [SEQ ID NO: 3]

In one embodiment, the kit can comprise a probe. Examples of probe sequences that can be used to detect the internal control nucleic acid include, but are not limited to, the following:
Cy5- TATCTTGCAC CGTTCAGCCA TCTC-BHQ-2 [SEQ ID NO: 4]

### [Examples]

Having now broadly described the invention, it is believed that the same will be more readily understood by reference to the following examples. However, the examples are provided for illustrative purposes only and are not intended to limit the invention.

### [Manufacture Example]

### [Preparation of internal control nucleic acid]

Although the method for preparing the internal control nucleic acid is not limited to these, the internal control nucleic acids used in the examples were prepared as follows.

### [Comparative Example]

### Design example of random base sequence of approximately 1,000 bases (A)

Blastn was performed approximately 6,000 times, and multiple artificial sequences of approximately 30 bases in length with low homology to known sequences were obtained. By combining these multiple sequences, a sequence of approximately 1000 bases was designed. When BLAST was performed on the sequence of approximately 1,000 bases long designed using this procedure, a sequence with high homology to the known sequence was created at the junction, and no sequence with an E-value>30 was obtained. In conclusion, this procedure did not result in the acquisition of a sequence of approximately 1000 bases with sufficiently low homology to known sequences.

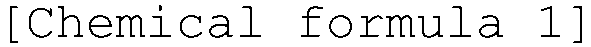

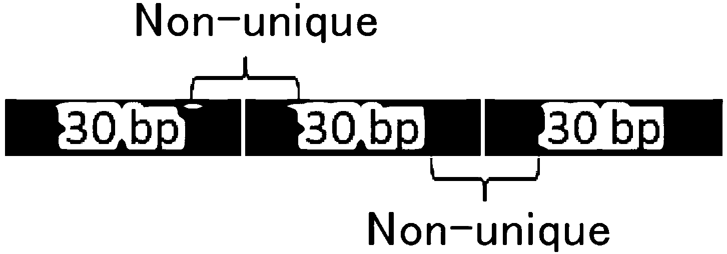

### Design example of random base sequence of approximately 1000 bases (B) manufacture example

Blastn was performed approximately 6,000 times, and artificial sequences of approximately 1000 bases in length with low homology to known sequences were obtained. Of the 6000 sequences obtained, approximately 10 had an E-value>30. However, even in the sequence with the highest E-value obtained, there were still regions with relatively high homology to known sequences; therefore, regions with lower homology to known sequences were extracted from the two sequences with the highest E-value and bound. Furthermore, while checking whether the sequence was unique using blastn, text editing software was used to correct for 5 or more consecutive bases and bias in the GC content, adjusting the content to 40% to 60%. The resulting base sequence (designated SEQ ID NO: 1) was subjected to blastn; among the multiple sequences obtained, the number of examples of the sequence with the highest E-value was 348 (as of November 7, 2024). In conclusion, this procedure allowed us to obtain a sequence of approximately 1000 bases with sufficient low homology to known sequences.

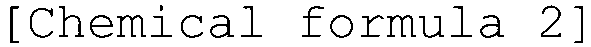

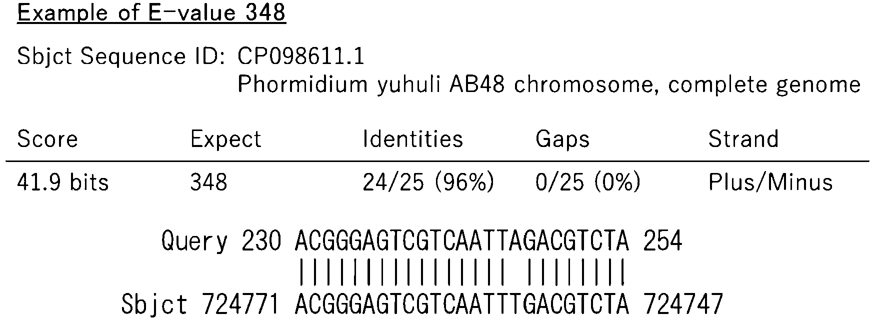

The DNA shown in SEQ ID NO: 1 was actually synthesized using an artificial gene synthesis service. The DNA was delivered in a form integrated into a vector, and only the region of sequence 1 was amplified using PCR. Furthermore, the DNA of SEQ ID NO: 1 was purified from the reaction solution of after amplification to prepare an internal control nucleic acid.

### [Preparation of primers and probes]

A forward primer for detecting an internal control nucleic acid shown in SEQ ID NO: 2, a reverse primer for detecting an internal control nucleic acid shown in SEQ ID NO: 3, and a probe (Cy5-labeled) for detecting an internal control nucleic acid shown in SEQ ID NO: 4 were synthesized using an oligo DNA synthesis service.

### [Example 1]

### Usage of internal control nucleic acids in gene detection of microbial samples

### 1. Materials and methods

### [Preparation of bacterial genomic DNA]

*Pseudomonas aeruginosa* (carrying the blaVIM gene) and *Escherichia coli* (not carrying the blaVIM gene) strains were cultured on SCD agar medium. Genomic DNA was purified from the cultured cells using a column purification method, and the DNA was adjusted to 1 ng/µL with TE buffer solution to serve as a sample. TE buffer solution was used as a negative control.

### Sequences of primers and probes used

Forward primer for detecting *bla*VIM gene
   GCTTCGGTCC AGTAGA [SEQ ID NO: 5]
Reverse primer for detecting *bla*VIM gene
   GTTGTGTACG TCCCGTCTGC [SEQ ID NO: 6]
Probe for detecting *bla*VIM gene (VIC labeled)
   VIC-TTCTATCCTG GTGCTGCGCA TTCG-BHQ-1 [SEQ ID NO: 7]

### Reaction solution

Using Roche's AptaTaq DNA Master (5x Conc.), 20 µL of PCR reaction solution containing the following reagents was prepared.
Sample (1 ng/µL) or negative control: 5 µL
Forward primer for detecting *bla*VIM gene: 400 nM
Reverse primer for detecting *bla*VIM gene: 400 nM
Forward primer for detecting internal control nucleic acid: 400 nM
Reverse primer for detecting internal control nucleic acid: 400nM
Probe for detecting *bla*VIM gene (VIC labeled): 100 nM
Probe for detecting Internal control nucleic acid (Cy5 labeled): 40 nM
AptaTaq DNA Master (5×Conc.): 4 µL
Internal control nucleic acid: 2.5 fg

### [Real-time PCR]

The prepared reaction solution was subjected to a real-time PCR machine (QuantStudio 5 Dx) manufactured by Thermo Fisher Scientific, and real-time PCR was carried out under the following conditions. Real-time PCR was performed according to the machine's instruction manual.

### Nucleic acid amplification and fluorescence detection conditions by real-time PCR method

(i) 94°C for 15 seconds
(ii) 60°C for 60 seconds (fluorescence detection)

### (35 cycles of (i) and (ii))

### Capillary electrophoresis

The reaction solution after real-time PCR was subjected to capillary electrophoresis using a QIAGEN's capillary electrophoresis apparatus (QIAxcel Advanced System) and the following reagents. Capillary electrophoresis was performed according to the apparatus's instruction manual.
Gel Cartridge: QIAxcel DNA High Resolution Kit
QX Alignment Marker: QX Alignment Marker 15 bp/3 kb
QX DNA Size Marker: QX DNA Size Marker 50 - 800

### 2. Results

The results of real-time PCR are shown as graphs with the fluorescence intensity on the vertical axis and the cycle number on the horizontal axis; FIG. 1 shows the results for *Pseudomonas aeruginosa* (carrying the *bla*VIM gene), FIG. 2 shows the results for *Escherichia coli* (not carrying the blaVIM gene), and FIG. 3 shows the results for the negative control. In FIG. 1, an increase in the fluorescence intensity of VIC, which indicates that the *bla*VIM gene was amplified, and an increase in the fluorescence intensity of Cy5, which indicates that the internal control nucleic acid (IC) was amplified, were observed. In addition, in FIGs. 2 and 3, only an increase in the fluorescence intensity of Cy5 was observed, indicating that the internal control nucleic acid (IC) was amplified. These results indicate that the reaction was not inhibited by the sample and that the real-time PCR reaction was carried out normally. In other words, it is clear that the results in FIGs. 2 and 3, in which the *bla*VIM gene is determined to be negative, are not false negatives.

Furthermore, the reaction solution after real-time PCR was analyzed by capillary electrophoresis, and the results were plotted as a graph with the fluorescence intensity (RFU) on the vertical axis and the size (bp) on the horizontal axis. FIG. 4 shows the analysis results for *Pseudomonas aeruginosa* (carrying the *bla*VIM gene), FIG. 5 shows the analysis results for *Escherichia coli* (not carrying the *bla*VIM gene), and FIG. 6 shows the analysis results for the negative control. The expected sizes of the amplification products are 76 bp for *bla*VIM and 67 bp for the internal control nucleic acid. The 15 bp and 3000 bp peaks are alignment markers used in the capillary electrophoresis analysis, and do not indicate the presence of 15 bp and 3000 bp amplification products in the reaction solution. In FIG. 4, clear peaks were detected at 67 bp and 76 bp, which are the amplified sizes of the internal control nucleic acid and *bla*VIM, and no clear peaks of non-specific amplification products were detected. Although a peak was detected between 15 bp and 67 bp, this was a peak of primer dimer and did not represent a non-specific amplification product. In FIGs. 5 and 6, only a peak of about 67 bp, which is the amplified size of the internal control nucleic acid, was clearly detected, and no peaks of obvious non-specific amplification products were detected. These results demonstrated that the internal control nucleic acid, and the primers and probes for detecting the internal control nucleic acid can be used to detect the *bla*VIM gene from microorganisms. That is, the internal control nucleic acid of the present invention was demonstrated to be able to determine that the PCR result is not a false negative.

### [Example 2]

### Usage of internal control nucleic acids in gene detection in animal cell samples

### 1. Materials and methods

### [Preparation of mouse genomic DNA]

Genomic DNA was purified from BALB/c mouse hybridoma cells using a column purification method, and the DNA was adjusted to 100 ng/µL with TE buffer solution to serve as a sample. TE buffer solution was used as a negative control.

### Sequences of primers and probes used

Forward primer for detecting β-globin gene
   GTGAGCTCCA CTGTGACAAG [SEQ ID NO: 8]
Reverse primer for detecting β-globin gene
   CACACACCAT CATCGAAACT [SEQ ID NO: 9]
Probe for detecting β-globin gene (FAM-labeled)
   FAM-CTTCCCCTGG CTATTCTGCT CA-BHQ-1 [SEQ ID NO: 10]

### Reaction solution

Using Roche's AptaTaq DNA Master (5x Conc.), 20 µL of reaction solution containing the following reagents was prepared.
Sample (100 ng/µL) or negative control: 5 µL
Forward primer for detecting β-globin gene: 200 nM
Reverse primer for detecting β-globin gene: 200 nM
Forward primer for detecting internal control nucleic acid: 400 nM
Reverse primer for detecting internal control nucleic acid: 400nM
Probe for detecting β-globin gene (FAM-labeled): 100 nM
Probe for detecting internal control nucleic acid (Cy5 labeled): 40 nM
AptaTaq DNA Master (5×Conc. ): 4 µL
Internal control nucleic acid: 2.5 fg

### [Real-time PCR]

The prepared PCR reaction solution was subjected to a real-time PCR machine (QuantStudio 5 Dx) manufactured by Thermo Fisher Scientific, and real-time PCR was carried out under the following conditions. Real-time PCR was performed according to the machine's instruction manual.

### Nucleic acid amplification and fluorescence detection conditions by real-time PCR method

(i) 94°C for 15 seconds
(ii) 60°C for 60 seconds (fluorescence detection)
(40 cycles of (i) and (ii))

### Capillary electrophoresis

The reaction solution after real-time PCR was subjected to capillary electrophoresis using a QIAGEN's capillary electrophoresis apparatus (QIAxcel Advanced System) and the following reagents. Capillary electrophoresis was performed according to the apparatus's instruction manual.
Gel Cartridge: QIAxcel DNA High Resolution Kit
QX Alignment Marker: QX Alignment Marker 15 bp/3 kb
QX DNA Size Marker: QX DNA Size Marker 50 - 800

### 2. Results

The results of real-time PCR are shown as graphs with the fluorescence intensity on the vertical axis and the cycle number on the horizontal axis; FIG. 7 shows the results for BALB/c mouse hybridoma cells (carrying the β-globin gene), and FIG. 8 shows the results for the negative control. In FIG. 7, an increase in the fluorescence intensity of FAM, which indicates that the β-globin gene was amplified, and an increase in the fluorescence intensity of Cy5, which indicates that the internal control nucleic acid (IC) was amplified, were observed. In addition, in FIG. 8, only an increase in the fluorescence intensity of Cy5 was observed, indicating that the internal control nucleic acid (IC) was amplified. These results indicate that the reaction was not inhibited by the sample and that the real-time PCR reaction was carried out normally. In other words, it is clear that the results in FIG. 8, in which the β-globin gene is determined to be negative, are not false negatives.

Furthermore, the reaction solution after real-time PCR was analyzed by capillary electrophoresis, and the results were plotted as a graph with the fluorescence intensity (RFU) on the vertical axis and the size (bp) on the horizontal axis. FIG. 9 shows the analysis results of BALB/c mouse hybridoma cells (carrying the β-globin gene), and FIG. 10 shows the analysis results of the negative control. The expected sizes of the amplification products are 159 bp for the β-globin gene and 67 bp for the internal control nucleic acid. The 15 bp and 3000 bp peaks are alignment markers used in the capillary electrophoresis analysis, and do not indicate the presence of 15 bp and 3000 bp amplification products in the reaction solution. In FIG. 9, clear peaks were detected at 67 bp and 159 bp, which are the amplified sizes of the internal control nucleic acid and the β-globin gene, and no clear peaks of non-specific amplification products were detected. Although a peak was detected between 15 bp and 67 bp, this was a peak of primer dimer and did not represent a non-specific amplification product. In FIG. 10, only a peak of about 67 bp, which is the amplified size of the internal control nucleic acid, was clearly detected, and no peaks of obvious non-specific amplification products were detected. These results demonstrated that the internal control nucleic acid, and the primers and probes for detecting the internal control nucleic acid can be used to detect the β-globin gene from animal cells. That is, the internal control nucleic acid of the present invention was demonstrated to be able to determine that the PCR result is not a false negative.

### [Example 3]

### Usage of internal control nucleic acids in gene detection of viral samples

### 1. Materials and methods

### [Preparation of positive control nucleic acid]

An RS virus positive control nucleic acid of RNA shown in SEQ ID NO: 11 was prepared, and adjusted to 10 fg/µL with TE buffer solution to serve as a sample. TE buffer solution was used as a negative control.

### Sequences of RS virus positive control nucleic acid and primer and probe used

rs positive rna 
TAATACGACT CACTATAGAG GGTCATCGTC TTTTTCTAGG ACATTGTATT GAACAGCAGC 60
TGTGTATGTG GAGCCTTCGT GAAGCTTGTT CACGTATGTT TCCATATTTG CCCCACCCCT 120
CGAG 124

[SEQ ID NO: 11]
Forward primer for detecting RS virus
   GGCAAATATG GAAACATACG TGAA [SEQ ID NO: 12]
Reverse primer for detecting RS virus
   TCTTTTTCTA GGACATTGTA YTGAACAG [SEQ ID NO: 13]
Probe for detecting RS virus
   FAM-TCTTTTTCTA GGACATTGTA YTGAACAG-BHQ-1 [SEQ ID NO: 14]
(In the sequence, Y represents a mixed base of C and T.)

### Reaction solution

Using AgPath-ID (trade mark) One-Step RT-PCR Reagents manufactured by Thermo Fisher Scientific, 25 µL of RT-PCR solution containing the following reagents was prepared.
Sample (10 fg/µL) or negative control: 5 µL
Forward primer for detecting RS virus gene: 500 nM
Reverse primer for detecting RS virus gene: 300 nM
Forward primer for detecting internal control nucleic acid: 400 nM
Reverse primer for detecting internal control nucleic acid: 400 nM
Probe for detecting RS virus gene (FAM labeled): 150 nM
Probe for detecting internal control nucleic acid (Cy5 labeled): 40 nM
2X RT-PCR Buffer: 12.5 µL
25X RT-PCR Enzyme Mix: 1 µL
Internal control nucleic acid: 2.5 fg

### [Real-time RT-PCR]

The prepared RT-PCR reaction solution was subjected to a real-time PCR machine (QuantStudio 5 Dx) manufactured by Thermo Fisher Scientific, and real-time RT-PCR was carried out under the following conditions. Real-time RT-PCR was performed according to the machine's instruction manual.

### Nucleic acid amplification and fluorescence detection conditions by real-time RT-PCR method

(i) 48°C for 10 minutes
(ii) 95°C for 5 minutes
(iii) 95°C for 15 seconds
(iiii) 55°C for 60 seconds (fluorescence detection)

### ((iii) and (iiii) for 45 cycles)

### Capillary electrophoresis

The reaction solution after real-time RT-PCR was subjected to capillary electrophoresis using a QIAGEN's capillary electrophoresis apparatus (QIAxcel Advanced System) and the following reagents. Capillary electrophoresis was performed according to the apparatus's instruction manual.
Gel Cartridge: QIAxcel DNA High Resolution Kit
QX Alignment Marker: QX Alignment Marker 15 bp/3 kb
QX DNA Size Marker: QX DNA Size Marker 50 - 800

### 2. Results

The results of real-time RT-PCR are shown as graphs with the fluorescence intensity on the vertical axis and the cycle number on the horizontal axis; FIG. 11 shows the results for the RS virus positive control, and FIG. 12 shows the results for the negative control. In FIG. 11, an increase in the fluorescence intensity of FAM, indicating that the RS virus positive control was amplified, and an increase in the fluorescence intensity of Cy5, indicating that the internal control nucleic acid (IC) was amplified, were observed. In addition, in FIG. 12, only an increase in the fluorescence intensity of Cy5 was observed, indicating that the internal control nucleic acid (IC) was amplified. These results indicate that the reaction was not inhibited by the sample and that the real-time RT-PCR reaction was carried out normally. In other words, it is clear that the results in FIG. 12, in which the RS virus positive control was determined to be negative, are not false negatives.

Furthermore, the reaction solution after real-time RT-PCR was analyzed by capillary electrophoresis, and the results were plotted as a graph with the fluorescence intensity (RFU) on the vertical axis and the size (bp) on the horizontal axis. FIG. 13 shows the analysis results of the RS virus positive control, and FIG. 14 shows the analysis results of the negative control. The expected size of the amplification product is 84 bp for the RS virus positive control and 67 bp for the internal control nucleic acid. The 15 bp and 3000 bp peaks are alignment markers used in the capillary electrophoresis analysis, and do not indicate the presence of 15 bp and 3000 bp amplification products in the reaction solution. In FIG. 13, clear peaks were detected at 67 bp and 84 bp, which are the amplified sizes of the internal control nucleic acid and the RS virus positive control, and no clear peaks of non-specific amplification products were detected. Although a peak was detected between 15 bp and 67 bp, this was a peak of primer dimer and did not represent a non-specific amplification product. In FIG. 14, only a peak of about 67 bp, which is the amplified size of the internal control nucleic acid, was clearly detected, and no peaks of obvious non-specific amplification products were detected. These results demonstrated that the internal control nucleic acid and the primers and probes for detecting the internal control nucleic acid can be used to detect genes from viruses.

### [Industrial Applicability]

The internal control nucleic acid of the present invention has low homology to known nucleic acid sequences and can therefore be used in nucleic acid amplification methods to detect various genes regardless of the type of specimen (animal, plant, microorganism, virus), and it can determine whether or not there are false negatives, making it useful for quality control of genetic testing. Furthermore, whereas it has previously been necessary to design an internal control nucleic acid and a primer/probe for detecting the internal control nucleic acid for each specimen type and target gene to be detected, the present invention allows the internal control nucleic acid and the primer/probe for detecting the internal control nucleic acid to be used in common, which can contribute to rapid development of genetic testing kits and cost reduction.

[Sequence List]

## Claims

1. An internal control nucleic acid comprising a sequence that is at least 90% identical to a sequence of 50 or more consecutive bases selected from the base sequence shown in SEQ ID NO: 1.

2. The internal control nucleic acid according to claim 1, wherein the internal control nucleic acid comprises a sequence of 50 or more consecutive bases selected from the base sequence shown in SEQ ID NO: 1;
preferably wherein the internal control nucleic acid consists of a sequence of 50 or more consecutive bases selected from the base sequence shown in SEQ ID NO: 1; or more preferably wherein the internal control nucleic acid consists of the base sequence shown in SEQ ID NO: 1.

3. A composition for the detection and/or quantification of nucleic acids, containing:
an internal control nucleic acid according to claim 1 or 2;
a forward primer and a reverse primer for amplifying a part or all of the nucleic acid; and a sample,
preferably wherein the composition further containing an oligonucleotide probe complementary to an internal control nucleic acid in the region flanked by the forward primer and the reverse primer.

4. The composition according to claim 3, wherein
(a) the oligonucleotide of the forward primer is at least 95% identical to the base sequence shown in SEQ ID NO: 2;
(b) the oligonucleotide of the reverse primer is at least 95% identical to the base sequence shown in SEQ ID NO: 3; and/or
(c) the oligonucleotide probe is at least 95% identical to the base sequence shown in SEQ ID NO: 4.

5. A method for detecting and/or quantifying a nucleic acid in a sample, comprising:
a) a step of mixing an internal control nucleic acid comprising a sequence that is at least 90% identical to 50 or more consecutive bases selected from the base sequence shown in SEQ ID NO: 1 with a forward primer and a reverse primer for amplifying a part or all of the nucleic acid, and a sample;
b) a step of extending the forward and reverse primers, thereby generating at least one target amplicon; and
c) a step of detecting and/or quantifying the amplification of the target amplicon by electrophoresis.

6. A method for detecting and/or quantifying a nucleic acid in a sample, comprising:
a) a step of mixing an internal control nucleic acid comprising a sequence that is at least 90% identical to 50 or more consecutive bases selected from the base sequence shown in SEQ ID NO: 1 with a forward primer and a reverse primer for amplifying a part or all of the nucleic acid, an oligonucleotide probe, and a sample;
b) a step of extending the forward and reverse primers, thereby generating at least one target amplicon;
c) a step of binding the oligonucleotide probe to said at least one internal control nucleic acid or target amplicon; and
d) a step of detecting a signal proportional to the amount of said at least one internal control nucleic acid or target amplicon.

7. The method according to claim 6, wherein the internal control nucleic acid consists of a sequence of 50 or more consecutive bases selected from the base sequence shown in SEQ ID NO: 1, preferably wherein the internal control nucleic acid comprises a sequence consisting of the base sequence shown in SEQ ID NO: 1.

8. The method according to claim 5 or 6, wherein
(a) the oligonucleotide of the forward primer is at least 95% identical to the base sequence shown in SEQ ID NO: 2;
(b) the oligonucleotide of the reverse primer is at least 95% identical to the base sequence shown in SEQ ID NO: 3; or
(c) the oligonucleotide of the forward primer is the base sequence shown in SEQ ID NO: 2, and the oligonucleotide of the reverse primer is the base sequence shown in SEQ ID NO: 3.

9. The method according to claim 5 or 6, wherein the internal control nucleic acid comprises a sequence of 50 or more consecutive bases selected from the base sequence shown in SEQ ID NO: 1, preferably wherein the internal control nucleic acid consists of a sequence of 50 or more consecutive bases selected from the base sequence shown in SEQ ID NO: 1.

10. The method according to claim 5 or 6, wherein the oligonucleotide probe is the base sequence shown in SEQ ID NO: 4.

11. The method according to claim 5 or 6, further comprising a step of extracting a nucleic acid from the sample before or after step a).

12. A method for designing an internal control nucleic acid in the detection and/or quantification of a nucleic acid, comprising:
a) a step of generating a random base sequence having a length of 500 to 1500 bases; and
b) a step of comparing the random base sequence with a database to select sequences with low homology, wherein the comparison with the database is performed using BLAST, and the E-value of the BLAST selection criteria is E-value>30, preferably wherein the method further comprising
a step of joining regions with high E-values from one of the base sequences selected in step b), or
a step of joining regions with high E-values from the two or more base sequences selected in b), or
preferably wherein the method further comprising a step of removing a base sequence containing five or more consecutive identical bases from the base sequences selected in b).

13. The method according to claim 12, further comprising a step of adjusting the GC content of the base sequence selected in b) to 40 to 60%.

14. The method according to claim 12 or 13, wherein the designed internal control nucleic acid has an E-value of 300 or more by BLAST and a length of 500 to 1500 bases.

15. A method for designing an internal control nucleic acid in the detection and/or quantification of a nucleic acid, comprising:
a) a step of generating a random base sequence having a length of 500 to 1500 bases;
b) a step of selecting a sequence with low homology from the random base sequences by comparing with a database, wherein the comparison with the database is performed using BLAST, and the E-value of the BLAST selection criteria is E-value>30;
c) a step of removing a base sequence containing five or more consecutive identical bases from the base sequences selected in b);
d) a step of adjusting the GC content of the base sequence selected in b) to 40-60%; and/or
e) a step of further selecting a region with a high E-value from one base sequence selected in b) and joining the selected regions, or a step of selecting a region with a high E-value from two or more base sequences selected in b) and joining the selected regions;
wherein the designed internal control nucleic acid has an E-value of 300 or more by BLAST and a length of 500 to 1500 bases.
